(19) Europäisches Patentamt
European Patent Office
Office européen des brevets

(11) **EP 2 612 653 B2**

(12) **NEW EUROPEAN PATENT SPECIFICATION**
After opposition procedure

(45) Date of publication and mention
of the opposition decision:
**09.06.2021 Bulletin 2021/23**

(45) Mention of the grant of the patent:
**11.10.2017 Bulletin 2017/41**

(21) Application number: **11821532.6**

(22) Date of filing: **09.08.2011**

(51) Int Cl.:
*A61K 8/44* (2006.01)        *A61Q 5/02* (2006.01)
*C11D 1/10* (2006.01)        *C11D 1/66* (2006.01)
*C11D 1/88* (2006.01)        *C11D 3/37* (2006.01)
*C11D 1/94* (2006.01)        *A61Q 5/12* (2006.01)
*A61K 8/42* (2006.01)        *A61K 8/46* (2006.01)
*A61K 8/49* (2006.01)        *A61K 8/33* (2006.01)

(86) International application number:
**PCT/JP2011/068154**

(87) International publication number:
**WO 2012/029514 (08.03.2012 Gazette 2012/10)**

(54) **COMPOSITION FOR CLEANING SCALP AND HEAD HAIR**

ZUSAMMENSETZUNG ZUR REINIGUNG VON KOPFHAUT UND KOPFHAAR

COMPOSITION POUR LE NETTOYAGE DE LA TÊTE ET DES CHEVEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.08.2010 JP 2010194281**

(43) Date of publication of application:
**10.07.2013 Bulletin 2013/28**

(73) Proprietor: **Otsuka Pharmaceutical Co., Ltd.**
**Tokyo 101-8535 (JP)**

(72) Inventors:
• **OGIHARA, Miyoko**
**Osaka-shi**
**Osaka 541-0045 (JP)**
• **SHIOYAMA, Momoko**
**Osaka-shi**
**Osaka 541-0045 (JP)**
• **TANAKA, Masahiko**
**Osaka-shi**
**Osaka 541-0045 (JP)**
• **YOSHINO, Noboru**
**Osaka-shi**
**Osaka 541-0045 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
EP-A1- 0 648 833        EP-A1- 1 925 290
EP-A1- 2 044 925        EP-A2- 0 155 737
WO-A1-99/24004        DE-A1- 10 218 302
JP-A- 2001 181 354        JP-A- 2001 181 354
JP-A- 2005 307 019        JP-A- 2005 350 645
JP-A- 2006 348 101        JP-A- 2006 348 101
JP-A- 2008 024 628        JP-A- 2008 024 628

• **ASAHI FOODS KABUSHIKI KAISHA: 'Amino Acid-kei Kaimen Kasseizai 'Aminosafakuto''** FRAGRANCE JOURNAL vol. 18, no. 5, 15 May 1990, page 79, XP008169149
• **HIROYUKI ARAI ET AL.: 'Performance and features of sodium lauroyl aspartate'** FRAGRANCE JOURNAL vol. 36, no. 7, 15 August 2008, pages 43 - 48, XP008169155
• **ASAHI KASEI CHEMICALS CORP.: 'Aminofoma FLDS-L' FRAGRANCE JOURNAL** vol. 33, no. 2, 15 February 2005, page 120, 121, XP008169150

Remarks:
The file contains technical information submitted after the application was filed and not included in this specification

**Description**

TECHNICAL FIELD

The present invention relates to a composition for cleaning (washing) scalp and head hair with an excellent feeling in use, which has a suitable viscosity and provides a conditioning benefit.

BACKGROUND ART

In order to improve the conditioning performance of a hair cosmetic preparation such as a shampoo, various approaches have been done. As examples having such performance, hair cosmetic preparations comprising an anionic surfactant, a water-soluble resin, and a non-ionic surfactant and/or an ampholytic surfactant are known (see, Patent reference 1).

An acylamino acid type surfactant is a useful surfactant because it is less irritating and thereby it can clean skin and hair without damaging them, thus the acylamino acid type surfactant is used in a cleaning agent for body or hair (see, Patent reference 2).

Generally, a cleaning agent such as a body shampoo and a hair shampoo is required to have a certain level of viscosity because it is used after taking it out from a container thereof onto a palm. However, it is also known that the viscosity of a hair cleaning agent is reduced by using a high concentration of such acylamino acid type surfactant.

A cleaning composition with moderate viscosity comprising an acylamino acid type surfactant in combination with a thickening agent having at least one ethylene oxide structure is known from patent reference 3.

Hitherto, a thickener such as guar gum has been added thereto as a way to improve the low viscosity. But, it is known that some thickeners such as polysaccharides have problems such as color change (for example, white turbidity), and precipitation, if they are used.

It is known that an anionic surfactant is used in combination with a non-ionic surfactant in order to increase the viscosity of a cleaning agent while preventing said problem caused by the addition of thickeners such as color change and precipitation. However, it is difficult to increase the viscosity if an anionic surfactant of acylamino acid type is used as a main cleaning ingredient, because the addition of a non-ionic ingredient is unhelpful to increase the viscosity of such cleaning agent.

Due to the problem as mentioned above that a cleaning agent which contains an acylamino acid type surfactant as a main cleaning ingredient is not so easy to use because of the low viscosity, in fact, the use of an acylamino acid type surfactant is limited to a case of a low concentration of an acylamino acid type surfactant. As a result, the conditioning benefit provided by an acylamino acid type surfactant is not enough exerted despite its potential of conditioning benefit. Accordingly, it is tried to improve the conditioning performance by adding additional ingredients such as silicone, but silicone has a problem that it accumulates on hair/scalp and results in an oily feeling.

In addition, when an acylamino acid type surfactant is used in a cleaning composition in a high concentration, it has a disadvantage of reducing lather generation. There has not been known any cleaning composition comprising a high concentration of acylamino acid type surfactant which has a suitable viscosity for use and has an excellent quick-foaming property.

[Patent Reference]

   Patent reference 1: WO2005/074868
   Patent reference 2: JP 2006-306908 A
   Patent reference 3: JP 2006-348101 A

SUMMARY OF INVENTION

(PROBLEMS TO BE SOLVED BY INVENTION)

As described above, although an acylamino acid type surfactant is suitable for scalp/head hair application because it causes little irritation of skin, it is difficult to increase the viscosity of a hair formulation comprising an acylamino acid type surfactant compared with other commonly-used surfactants, and hence, it has been difficult to use a high concentration of an acylamino acid type surfactant in a cleaning agent such as a shampoo. The present invention provides a cleaning composition which comprises a high concentration of an acylamino acid type surfactant while keeping a suitable viscosity. It is also a purpose of the present invention to provide a cleaning composition comprising a high concentration of an acylamino acid type surfactant which additionally has an excellent conditioning performance.

**(MEANS TO SOLVE PROBLEMS)**

The present inventors have extensively studied to find out a solution of said problems, and then they have found that the use of a certain range of the amounts of a particular ampholytic surfactant and a non-ionic surfactant enables a cleaning composition comprising a high concentration of an acylamino acid type surfactant with a suitable viscosity, even without a thickener. They have completed a cleaning composition which is less irritating to scalp and additionally has an excellent protective effect on hair by using a cationic polymer in addition to the above-mentioned three ingredients. That is, the present invention provides a cleaning agent, especially those available as a shampoo or a conditioning shampoo, which provides users with a good usability and a good feeling in use while having a conditioning benefit even without silicone.

Specifically, the present invention relates to a cleaning composition which comprises: 7.5 to 12 % by weight of a specific acylamino acid type anionic surfactant, which is a higher amount compared to the usual case; a betaine-type ampholytic surfactant; and 1.5 to 10 % by weight of a non-ionic surfactant, and which cleaning composition has a viscosity of 300 to 4000 mPa · s at 20 °C. Moreover, the coacervate-forming ability of the cleaning composition is enhanced by adding a cationic polymer, and thereby the obtained cleaning composition has an excellent conditioning performance (for example, repairing damaged hair).

The present invention has been completed by further studying on the basis of these findings.

That is, the present invention provides the following aspects.

Aspect 1. A cleaning composition applicable to scalp and head hair which comprises

Ingredient (a): an acylamino acid type surfactant 7.5 to 12 % by weight;
Ingredient (b): a betaine-type ampholytic surfactant; and
Ingredient (c): a non-ionic surfactant 1.5 to 10 % by weight,
wherein the acylamino acid type surfactant of Ingredient (a) is a compound represented by the formula:

$$R-NH-CH(COOX_1)((CH_2)_nCOOX_2)$$

wherein

R is an acyl group having 8 to 20 carbon atoms;
n is 1; and
$X_1$ and $X_2$ are each independently a hydrogen atom, an alkali metal, an alkaline earth metal, an ammonium group, or a cationic residue of a basic amino acid or an alkanolamine,
wherein the betaine-type ampholytic surfactant of Ingredient (b) is amide carboxy betaine;
wherein the non-ionic surfactant of Ingredient (c) is selected from POE glycerin fatty acid ester, POE sorbitan fatty acid ester, POE sorbitol fatty acid ester, ethylene glycol fatty acid ester, propylene glycol fatty acid ester, butylene glycol fatty acid ester, pentaerythritol fatty acid ester, and an ether type non-ionic surfactant which is in the form of a condensation product of alkyl glycol and propylene glycol, wherein the amount of Ingredient (b) is from 0.4 to 1 times by weight as much as the amount of Ingredient (a); wherein the amount of Ingredient (b) is from 1.5 to 3 times by weight as much as the amount of Ingredient (c); wherein the amount of Ingredient (c) is from 0.2 to 0.5 times by weight as much as the amount of Ingredient (a), and
wherein the cleaning composition has a viscosity ranging from 300 to 4000 mPa · s at 20°C.

Aspect 2. The cleaning composition according to Aspect 1, wherein R is selected from a capryloyl group, a caprinoyl group, a lauroyl group, a myristoyl group, a palmitoyl group, a stearoyl group, an oleoyl group, a cocoyl group, an acyl group derived from palm kernel oil fatty acid, and an acyl group derived from hydrogenated tallow fatty acid.
Aspect 3. The cleaning composition according to Aspect 1 or 2, wherein the acylamino acid type surfactant of Ingredient (a) is lauroyl aspartic acid or a salt thereof.
Aspect 4. The cleaning composition according to any one of Aspects 1-3, which comprises 2 to 5 % by weight of the non-ionic surfactant of Ingredient (c).
Aspect 5. The cleaning composition according to any one of Aspects 1-4, which further comprises Ingredient (d): a cationic polymer, wherein the amount of Ingredient (d) is from 0.025 to 1 times by weight as much as the amount of Ingredient (a).

Aspect 6. The cleaning composition according to Aspect 5, wherein the cationic polymer of Ingredient (d) comprises cationic cellulose and/or dimethyldiallyl ammonium chloride • acrylamide copolymer.

Aspect 7. The cleaning composition according to any one of Aspects 1-6, which further comprises Ingredient (e): an anionic surfactant other than an acylamino acid type surfactant 0.1 to 5 % by weight wherein said acylamino acid type surfactant is a compound represented by the formula:

$$R\text{-}NH\text{-}CH(COOX_1)((CH_2)_nCOOX_2)$$

wherein

R is an acyl group having 8 to 20 carbon atoms;
n is 1 or 2; and
$X_1$ and $X_2$ are each independently a hydrogen atom, an alkali metal, an alkaline earth metal, an ammonium group, or a cationic residue of a basic amino acid or an alkanolamine.

Aspect 8. The cleaning composition according to Aspect 7, wherein the anionic surfactant of Ingredient (e) is selected from:

an anionic surfactant which has an acyl group having 6 to 20 carbon atoms and a taurine skeleton;
an anionic surfactant which has an acyl group having 6 to 20 carbon atoms and an alanine skeleton; and
an anionic surfactant which is a sulfonic acid compound having an alkyl group and/or an alkenyl group each of which has 10 to 20 carbon atoms, or a salt thereof.

Aspect 9. The cleaning composition according to Aspect 1, which further comprises

Ingredient (d): a cationic polymer; and
Ingredient (e): an anionic surfactant other than an acylamino acid type surfactant which is a compound represented by the formula:

$$R\text{-}NH\text{-}CH(COOX_1)((CH_2)_nCOOX_2)$$

wherein

R is an acyl group having 8 to 20 carbon atoms;
n is 1 or 2; and
$X_1$ and $X_2$ are each independently a hydrogen atom, an alkali metal, an alkaline earth metal, an ammonium group, or a cationic residue of a basic amino acid or an alkanolamine, wherein the amount of Ingredient (d) is from 0.025 to 1 times by weight as much as the amount of Ingredient (a); and
the amount of Ingredient (e) is from 0.01 to 0.6 times by weight as much as the amount of Ingredient (a).

Aspect 10. The cleaning composition according to Aspect 9, wherein a coacervate is formed when the composition is diluted by 5 to 100-fold with water.

Aspect 11. The cleaning composition according to Aspect 1, which further comprises
Ingredient (e): an anionic surfactant 1 to 4 % by weight,
wherein the anionic surfactant of Ingredient (e) is selected from:

an anionic surfactant which has an acyl group having 6 to 20 carbon atoms and a taurine skeleton;
an anionic surfactant which has an acyl group having 6 to 20 carbon atoms and an alanine skeleton; and
an anionic surfactant which is a sulfonic acid compound having an alkyl group and/or an alkenyl group each of which has 10 to 20 carbon atoms, or a salt thereof,
wherein
the amount of Ingredient (b) is from 2 to 15 % by weight; and the amount of Ingredient (e) is from 0.1 to 0.5 times by weight as much as the amount of Ingredient (a).

## EFFECTS OF THE INVENTION

The cleaning (washing) composition of the present invention has a suitable viscosity though it comprises a high concentration of an acylamino acid type surfactant. The present invention can also provide a composition for cleaning scalp and head hair which has an excellent conditioning performance.

Specifically, the cleaning composition of the present invention comprises a betaine-type ampholytic surfactant and a non-ionic surfactant together with a given amount of an acylamino acid type surfactant, and thereby the present invention can provide a composition which has a suitable viscosity for use as a cleaning composition, causes little irritation of scalp, and provides a conditioning benefit. Moreover, the use of a cationic polymer in the composition enhances a coacervate formation, which may further improve smoothness of hair such as a smooth texture of hair in cleaning and a good smoothness in finger-combing after dried.

## BRIEF DESCRIPTION OF DRAWINGS

Figure 1 is a graph as to Test example 5-1 showing a change of the coacervate formation in relation to the dilution of a washing liquid which comprises a cationic polymer besides an acylamino acid type surfactant, a betaine-type ampholytic surfactant and a non-ionic surfactant.

Figure 2 is a graph as to Test example 5-2 showing a change of the coacervate formation in relation to the dilution of a washing liquid which comprises more than one kinds of cationic polymer besides an acylamino acid type surfactant, a betaine-type ampholytic surfactant, an non-ionic surfactant.

Figure 3 depicts electron microscope photographs of hair surfaces as to Test example 7 which show a contrast between before and after treating damaged hair with Washing liquid 10.

## DESCRIPTION OF EMBODIMENTS

Next, embodiments of the present invention are shown below.

The cleaning composition of the present invention is characterized by comprising: a 5 to 20 % by weight acylamino acid type surfactant; a betaine-type ampholytic surfactant; and a non-ionic surfactant.

The cleaning composition of the present invention includes, for example, what are used for cleaning scalp and/or head hair, or a whole body. Examples of the cleaning composition of the present invention include, in particular, a shampoo or a conditioning shampoo for scalp/head hair. In addition, the cleaning composition of the present invention may be used as a shampoo for a whole body.

The acylamino acid type surfactant used in the present invention is a surfactant having an acylamino acid moiety, and includes, for example, an anionic surfactant having an acylamino acid moiety.

In addition, the acylamino acid type surfactant used in the present invention may be a surfactant having two or more amino acid moieties wherein each of the amino acid moieties may be the same or different. The acylamino acid type surfactant used in the present invention may comprise two or more different acylamino acid type surfactants.

The acyl group of the acylamino acid type surfactant refers to a carbonyl group having a straight- or branched-chain saturated or unsaturated hydrocarbon group.

The acyl group includes, for example, acyl groups derived from caproic acid, caprylic acid, capric acid, lauric acid, myristic acid, palmitic acid, stearic acid, arachidic acid, palmitoleic acid, oleic acid, linoleic acid, linolenic acid, coconut oil fatty acid, castor oil fatty acid, olive oil fatty acid, palm oil fatty acid, palm kernel oil fatty acid and hydrogenated tallow fatty acid.

The number of carbon atoms of the acyl group is not limited, but an acyl group having 8 to 20 carbon atoms is preferable. From the viewpoint of an effect such as a smoothness in finger-combing after cleaning, an acyl group having 10 to 15 carbon atoms is preferable.

Examples of the acylamino acid type surfactant include lauroyl aspartic acid, cocoyl glutamic acid, myristoyl glutamic acid, coconut oil fatty acid acyl glutamic acid, lauroyl methyl alanine, cocoyl sarcosine, and cocoyl glycine, and a salt thereof.

Examples of the salt includes a metal salt such as sodium salt, potassium salt, lithium salt, magnesium salt, and calcium salt, an ammonium salt, an amine salt, a basic amino acid salt and a choline salt.

The preferred examples of an amino acid moiety comprised in an acylamino acid type surfactant include an acidic amino acid moiety such as aspartic acid and glutamic acid.

More preferred examples of the acylamino acid type surfactant include lauroyl aspartic acid, and cocoyl glutamic acid, and a salt thereof, which are available as AminoFoamer™ or Aminosurfact™ (Asahi Kasei Chemicals).

The most preferred examples of the anionic surfactant which has an acyl group having 10 to 15 carbon atoms and an acidic amino acid moiety used in the present invention include lauroyl aspartic acid, and cocoyl glutamic acid, and a salt thereof.

Preferred examples of the acylamino acid type surfactant include di-TEA-palmitoyl aspartate, diethyl palmitoyl aspartate, sodium lauroyl aspartate, zinc lauroyl aspartate, disodium capryloyl glutamate, cocoyl glutamic acid, disodium cocoyl glutamate, potassium cocoyl glutamate, sodium cocoyl glutamate, TEA-cocoyl glutamate, magnesium di-lauroylglutamate, sodium dilauramidoglutamide lysine, stearoyl glutamic acid, disodium stearoyl glutamate, aluminum stearoyl glutamate, potassium stearoyl glutamate, sodium stearoyl glutamate, dioctyldodecyl stearoyl glutamate, magnesium palmitoyl glutamate, myristoyl glutamic acid, potassium myristoyl glutamate, sodium myristoyl glutamate, lauroyl glutamic acid, disodium lauroyl glutamate, potassium lauroyl glutamate, sodium lauroyl glutamate, and TEA-lauroyl glutamate.

The acylamino acid type surfactant used in the present invention may comprise two or more different acylamino acid type surfactants.

The acylamino acid type surfactant is a compound represented by the formula:

$$R-NH-CH(COOX_1)((CH_2)_nCOOX_2)$$

wherein

R is an acyl group having 8 to 20 carbon atoms, preferably having 10 to 15 carbon atoms;
n is 1;
$X_1$ and $X_2$ are each independently a hydrogen atom, an alkali metal (for example, sodium or potassium), an alkaline earth metal (for example, calcium or magnesium), an ammonium group, or a cationic residue of a basic amino acid (for example, lysine or arginine) or an alkanolamine (for example, diethanolamine or triethanolamine).

Preferred examples of the above R include a capryloyl group, a caprinoyl group, a lauroyl group, a myristoyl group, a palmitoyl group, a stearoyl group, an oleoyl group, a cocoyl group, an acyl group derived from palm kernel oil fatty acid, and an acyl group derived from hydrogenated tallow fatty acid.

Examples of the above R having 10 to 15 carbon atoms include a caprinoyl group, a lauroyl group, a myristoyl group, and a cocoyl group.

The cleaning composition of the present invention comprises the acylamino acid type surfactant in the range of from 5 to 20 % by weight, preferably from 7 to 15 % by weight, and more preferably from 7.5 to 12 % by weight.

The ampholytic surfactant used in the present invention is a surfactant having both of a cation (cation group) an anion (anion group), notably isan ampholytic surfactant having a betaine skeleton which may be substituted partially (hereinafter referred to as "betaine-type ampholytic surfactant").

The betaine-type ampholytic surfactant is an:

amide carboxy betaine such as lauramidopropyl dimethyl betaine, myristamidopropyl dimethyl betaine, stearamidopropyl dimethyl betaine, oleamidopropyl dimethyl betaine, cocamidopropyl dimethyl betaine and lauramidopropyl betaine.

Preferred examples of the betaine-type surfactant used in the present invention include a betaine-type surfactant which has a betaine skeleton and a fatty acid moiety having 10 to 18 carbon atoms. If the fatty acid moiety has less than 10 carbon atoms, such betaine-type surfactant may cause an irritation. If the fatty acid moiety has more than 18 carbon atoms, such betaine-type surfactant may reduce lathering.

The betaine-type ampholytic surfactant used in the present invention may comprise two or more different betaine-type ampholytic surfactants.

The cleaning composition of the present invention may comprise a betaine-type ampholytic surfactant in the range of from 1 to 20 % by weight, preferably from 2 to 15 % by weight, and more preferably from 3 to 10 % by weight.

The non-ionic surfactant used in the present invention is selected from POE glycerin fatty acid ester (for example, POE glyceryl monococoate), POE sorbitan fatty acid ester (for example, POE sorbitan triisostearate), POE sorbitol fatty acid ester, ethylene glycol fatty acid ester, propylene glycol fatty acid ester, butylene glycol fatty acid ester (for example, butylene glycol laurate), pentaerythritol fatty acid ester, and an ether type non-ionic surfactant which is in the form of a condensation product of alkyl glycol and propylene glycol.

Such condensation product of alkyl glycol and propylene glycol includes, for example, lauryl glycol hydroxypropyl ether or myristyl glycol hydroxypropyl ether.

The non-ionic surfactant used in the present invention may comprise two or more different non-ionic surfactants.

The cleaning composition of the present invention comprises a non-ionic surfactant in the range of from 1.5 to 10 % by weight, preferably from 2 to 5 % by weight.

The composition of the present invention has a suitable viscosity for cleaning. The composition of the present invention has a viscosity ranging from 300 to 4000 mPa · s at 20°C, preferably ranging from 400 to 3000 mPa · s. The composition of the present invention having such viscosity may be in a suitable state for using as a body shampoo and a shampoo which are used after taking it out from a container thereof onto a palm.

A method to determine a viscosity is not limited, but includes, for example, a method using a B type viscometer with a rotor (No.2 or No.3) at a rate of 12 or 30 rpm at 20°C. Other examples of the method to determine a viscosity include ways specified in Japanese Pharmacopoeia (General Tests, Viscosity Determination; Method II viscosity measurement by rotational viscometer, Single cylinder-type rotational viscometer, 20°C, 12 or 30 rpm, Rotor No.2 or No.3).

In the cleaning composition of the present invention, the amount of a betaine-type ampholytic surfactant is from 0.4 to 1 times by weight as much as the amount of an acylamino acid type surfactant.

In the cleaning composition of the present invention, the amount of a non-ionic surfactant is from 0.2 to 0.5 times, by weight as much as the amount of an acylamino acid type surfactant.

In the cleaning composition of the present invention, the amount of a betaine-type ampholytic surfactant is from 1.5 to 3 times, by weight as much as the amount of a non-ionic surfactant, in order to give the above mentioned viscosity.

The coacervate-forming ability of the composition of the present invention may be enhanced by adding Ingredient (d) : a cationic polymer compound, and thereby the obtained composition may have an improved hair conditioning performance and may improve the feeling after cleaning.

Examples of the cationic polymer used in the present invention include any cationic polymer having a cationic moiety, and include for example, cationic cellulose such as 0-[2-hydroxy-3-(trimethylammonio)propyl]hydroxyethylcellulose chloride and [hydroxy(trimethylammonio)propyl]hydroxyethylcellulose chloride, a cationic hydrolyzed protein, a cationic vinyl type or acrylic type polymer (for example, dimethyldiallyl ammonium chloride · acrylamide copolymer), and a mixture thereof. Cationic cellulose, and a cationic vinyl type or acrylic type polymer are preferable.

Other examples of the cationic polymer include, for example, polyquaternium-4/hydroxypropyl starch copolymer, polyquaternium-10, polyquaternium-11, polyquaternium-64 and polyquaternium-7.

The cationic polymer used in the present invention may comprise two or more different cationic polymers.

In the cleaning composition of the present invention, preferably, the amount of a cationic polymer is adjusted according to the amount of an acylamino acid type surfactant from the viewpoints of the coacervate-forming ability, and the amount of a cationic polymer may be from 0.025 to 1 times, preferably 0.03 to 0.6 times, more preferably 0.04 to 0.5 times, by weight as much as the amount of an acylamino acid type surfactant.

The coacervate (or coacervation) as used herein is an insoluble complex which is formed by diluting a washing liquid such as shampoo which comprises a surfactant and a cationic polymer with water to lay the concentration in a particular range. The formed coacervate plays a role in a hair conditioning performance. For example, it is possible to reduce a friction between hair and fingers, and thereby it becomes possible to provide a good hair texture and a good feeling in finger-combing as well as an improved smoothness in finger-combing or combing after cleaning. The coacervate can remain after hair cleaning, especially even after rinsing and thereby it can bring in a hair conditioning benefit after cleaning.

The cleaning composition of the present invention may provide such coacervate benefits in various use situation, because the coacervate benefit is provided in cleaning scalp/head hair (the relative concentration of the cleaning composition during cleaning per its neat cleaning composition is usually in the range of 0.1 to 0.2), and in rinsing it (the relative concentration of the cleaning composition during rinsing per its neat cleaning composition is usually 0.1 or less).

The cleaning composition of the present invention may have pH which is usually available for scalp/head hair, however, it is preferable that the pH is in a particular range in order to improve a coacervate formation. The cleaning composition has preferably the pH being in the range of 4 to 8. In order to stabilize the viscosity of the cleaning composition, the cleaning composition more preferably has the pH being in the range of 4 to 6.5.

In addition, the composition of the present invention may further comprise Ingredient (e): an anionic surfactant in order to improve the quick-foaming property of an acylamino acid type surfactant. Preferably, said anionic surfactant is an anionic surfactant other than an acylamino acid type (Ingredient (a)) which has an amino acid moiety such as an aspartate moiety and a glutamate moiety. Examples of the anionic surfactant include lauroyl methyl taurine, lauroyl methyl alanine (lauroyl methyl beta-alanine), tetradecene sulfonic acid, cocoyl methyl taurine, cocoyl ethyl ester sulfonic acid, caproyl methyl taurine, and a salt thereof (for example, sodium salt, potassium salt, magnesium salt, or triethanolamine salt).

Furthermore, examples of Ingredient (e): an anionic surfactant includes:

an anionic surfactant which has an acyl group (preferably having 6 to 20 carbon atoms) and a taurine skeleton or an alanine skeleton, such as cocoyl methyl taurine, caproyl methyl taurine, lauroyl methyl alanine (lauroyl methyl beta-alanine) and a salt thereof, and
an anionic surfactant which is a sulfonic acid compound having an alkyl group and/or an alkenyl group each of which has 10 to 20 carbon atoms, and a salt thereof, such as alkylbenzene sulfonic acid, tetradecene sulfonic acid, and a salt thereof. The anionic surfactant used in the present invention may comprise two or more different anionic surfactants.

Examples of the anionic surfactant which has an acyl group and a taurine skeleton include a compound represented by the formula:

$$R\text{-}N(CH_3)((CH_2)_2SO_3X_1)$$

wherein

R is an acyl group having 6 to 20 carbon atoms,
$X_1$ is a hydrogen atom, an alkali metal (for example, sodium or potassium), an alkaline earth metal (for example, calcium or magnesium), an ammonium group, or a cationic residue of a basic amino acid (for example, lysine or arginine) or an alkanolamine (for example, diethanolamine or triethanolamine), and
the specific examples thereof include cocoyl methyl taurine and caproyl methyl taurine.

Said acyl group is the same as defined as to the acylamino acid type surfactant, and includes, for example, caproyl group, capryloyl group, caprinoyl group, lauroyl group, myristoyl group, palmitoyl group, stearoyl group, oleoyl group, cocoyl group, an acyl group derived from palm kernel oil fatty acid, and an acyl group derived from hydrogenated tallow fatty acid.

Examples of the anionic surfactant which has an acyl group and an alanine skeleton include a compound represented by the formula:

$$R\text{-}N(CH_3)((CH_2)_2COOX_1)$$

wherein

R is an acyl group having 6 to 20 carbon atoms,
$X_1$ is a hydrogen atom, an alkali metal (for example, sodium or potassium), an alkaline earth metal (for example, calcium or magnesium), an ammonium group, or a cationic residue of a basic amino acid (for example, lysine or arginine) or an alkanolamine (for example, diethanolamine or triethanolamine), and
the specific examples thereof include lauroyl methyl alanine (lauroyl methyl beta-alanine).

Said acyl group is the same as defined as to the acylamino acid type surfactant, and includes, for example, caproyl group, capryloyl group, caprinoyl group, lauroyl group, myristoyl group, palmitoyl group, stearoyl group, oleoyl group, cocoyl group, an acyl group derived from palm kernel oil fatty acid, and an acyl group derived from hydrogenated tallow fatty acid.

In the present invention, examples of the sulfonic acid compound having an alkyl group and/or an alkenyl group each of which has 10 to 20 carbon atoms include alkyl ($C_{10\text{-}14}$) benzene sulfonic acid, $\alpha$-olefin($C_{10\text{-}20}$) sulfonic acid, $\alpha$-olefin($C_{12\text{-}16}$) sulfonic acid, $\alpha$-olefin($C_{12\text{-}14}$) sulfonic acid, and $\alpha$-olefin($C_{14\text{-}16}$) sulfonic acid.

The composition of the present invention may comprise an anionic surfactant which is added in order to improve the quick-foaming property in the range of from 0.1 to 5 % by weight, preferably from 1 to 4 % by weight.

In the cleaning composition of the present invention, preferably, the amount of an anionic surfactant of Ingredient (e) which is added in order to improve the quick-foaming property may be from 0.01 to 0.6 times, more preferably from 0.1 to 0.5 times, by weight as much as the amount of an acylamino acid type surfactant of Ingredient (a).

The composition of the present invention may comprise an algefacient-ingredient in order to enhance a refreshing feeling without increasing an irritation. Said algefacient-ingredient may be a naturally-derived compound, a synthetic compound, and a salt thereof, and also the algefacient-ingredient may be any formation comprising these compounds. In addition, an algefacient-ingredient may be one algefacient-ingredient, or may be a combination of two or more algefacient-ingredients.

Preferred examples of the algefacient-ingredient which may be used in the composition of the present invention include menthol, and an essential oil, a plant-derived ingredient, which are capable of providing a cooling feeling.

The cleaning composition of the present invention may comprise an algefacient-ingredient preferably in the range of 0.1 % by weight or more, more preferably from 0.2 to 0.6 % by weight. If the amount of an algefacient-ingredient is more than 0.6 % by weight, the cleaning composition may cause an irritation of skin, and thereby the advantage of an acylamino acid type surfactant may be impaired.

The silicone-ingredient as described herein includes, for example, a polymer which has silicon oxide (SiO) as a basic skeleton and may have a hydrophilic substituent group. Examples of a silicone-ingredient include, for example, methylphenyl polysiloxane, methyl polysiloxane, octamethylcyclotetrasiloxane, decamethylcyclopentasiloxane, dodecamethylcyclohexasiloxane, methyl-cyclopolysiloxane, octamethyltrisiloxane, decamethyltetrasiloxane, polyoxyethylene · methylpolysiloxane copolymer, polyoxypropylene · methylpolysiloxane copolymer, poly(oxyethylene/oxypropylene) methylpolysiloxane copolymer, methylhydrogenpolysiloxane, tetra-hydrotetramethylcyclotetrasiloxane, stearoxymethylpolysiloxane, cetoxymethylpolysiloxane, methylpolysiloxane emulsion, highly polymerized methyl polysiloxane, trimethylsiloxysilicate, crosslinked methylpolysiloxane and crosslinked methylphenylpolysiloxane. Examples of the silicone-ingredient as described herein also include various derivatives such as an amino-modified silicone, an epoxy-modified silicone, a carboxyl-modified silicone, a carbinol-modified silicone, a methacryl-modified silicone, a mercapto-modified silicone, a phenol-modified silicone, a single-end reactive silicone, a co-modified silicone, a polyether-modified silicone, a methylstyryl-modified silicone, a alkyl-modified silicone, a higher fatty acid ester-modified silicone, a special hydrophilically-modified silicone, a higher alkoxy-modified silicone, a higher fatty acids-containing silicone and a fluorine-modified silicone.

In the present invention, "a silicone-ingredient is not comprised (without a silicone-ingredient) "means that a silicone-ingredient other than a left one due to a carryover or a left one in a storage solution is not comprised substantively. In addition, the "an effective amount of a silicone-ingredient is not comprised" also means that the concentration of a silicone-ingredient is not enough to show a conditioning benefit such as hair coating benefit.

The composition of the present invention may have the high transparency, because the viscosity of the composition may be increased even without a thickener (for example, cellulose gum, guar gum, gellan gum, sclerotium gum, cellulose gum, guar gum).

In order to prevent color change of the composition of the present invention due to oxidation during a long storage and in order to keep the composition transparent, the composition of the present invention may comprise an antioxidant such as sodium sulfite, and a color inhibitor at a concentration of, for example, from 0.01 to 0.5 % by weight. When the composition of the present invention has the high transparency, it is easy to color the composition, and the color of the composition may be changed to the desired color by adding a commercially available coloring agent.

The composition of the present invention may comprise a solvent which is available for a cleaning composition. Preferred examples of the solvent include water and dipropylene glycol.

Preferably, the concentration of dipropylene glycol lies in the range of from 0.5 to 5 % by weight.

When the composition of the present invention comprises water, a suitable amount of water may be used according to amounts of the other ingredients.

The composition of the present invention may comprise other ingredients which are usually used in a cleaning composition, besides the above ingredients. Examples of "ingredients which are usually used in a cleaning composition" as used herein include a surfactant, a polymer ingredient, a moisturizing agent, a preservative agent, a bactericidal agent such as isopropyl methylphenol, an anti-inflammatory agent such as glycyrrhizinic acid/glycyrrhizinate, an pH adjuster such as sodium hydroxide or citric acid, a sequestering agent such as EDTA, a tonicity agent and a flavor, in addition to the above-mentioned ingredients. In addition, the composition of the present invention may further comprise a conditioner-ingredient capable of protecting a surface of hair.

Moreover, the cleaning composition of the present invention may optionally include an active ingredient. The active ingredient as used herein refers to an ingredient which may provide scalp/head hair with a beneficial effect. Examples of the active ingredient include a hair growing agent, a hair regrowth agent, a deodorant and an anti-dandruff agent. Examples of the active ingredient include, for example, adenosine, adenosine phosphate, vitamins, minerals, edetic acid, milk-derived ingredients such as whey, carpronium chloride, matricaria oil, hinokitiol, benzylaminopurine, minoxidil, finasteride and a salt or derivative thereof, ginger, ginkgo, aloe, garlic, glycyrrhiza, chamomile, fennel, tincture, Swertia Herb, mulberry root bark, ginseng, moutan bark, Chinese Caterpillar Fungus, seaweed-derived ingredients such as fucoidan, Chitofilmer, salicylic acid and inositol.

EXAMPLES

The following Examples and Test examples serve to illustrate the present invention in more detail.

Unless otherwise specified, each amount of ingredients in the Examples is represented by % by weight (w/w %).

Test example 1: Test as to change in the feeling in use, in relation to the concentration of acylamino acid type surfactant

In order to evaluate the change in the feeling in use of a formulation for cleaning head hair in relation to the difference of acylamino acid type surfactants, the amount thereof, and a combination use thereof, cleaning formulations as showed in Table 1 were prepared, and then the sensory evaluation was done.

(Test method and Result)

Cleaning formulations as shown in Table 1 were prepared, and an appropriate amount of each cleaning formulation was applied onto the previously-wetted head hair and foamed. The feelings in use during and after cleaning head hair were evaluated (the amounts in the table are represented by a percentage by weight).

Table 1

| | Control 1 | Control 2 | Example 1 | Example 2 | Example 3 |
|---|---|---|---|---|---|
| Sodium Lauroyl Aspartate *1 | 3.8 | 3.8 | 7.5 | 8 | 8 |
| Sodium Cocoyl Glutamate *2 | | | | 0.9 | 0.9 |
| Sodium Methyl Cocoyl Taurate *3 | 3 | 3 | | 3.6 | 3.6 |
| Sodium Cocoyl Ethyl Ester Sulfonate *4 | | | | 0.5 | 0.5 |
| Sodium Tetradecenesulfonate *5 | 2 | 2 | 2 | | |
| Alkyl Carboxymethyl Hydroxyethyl Imidazolinium Betaine *6 | 6 | 6 | 4.5 | 2.1 | 6 |
| Lauramidopropyl Hydroxysultaine *7 | | | | 4 | |
| Lauryl Glycol Hydroxypropyl Ether *8 | | 2 | | 2 | 2 |
| POE Sorbitan Triisostearate *9 | | 1 | | 2 | 2 |
| Cationic Polymer | 0.6 | 1.1 | 0.6 | 0.8 | 0.8 |
| Moisturizing Agent · Preservative Agent · Bactericidal Agent · pH Adjuster etc. | Suitable Amount | Suitable Amount | Suitable Amount | Suitable Amount | Suitable Amount |
| Purified Water | Balance | Balance | Balance | Balance | Balance |
| Total | 100 | 100 | 100 | 100 | 100 |
| Feeling in use | Δ | Δ | ○ | ○ | ○ |

*1 : Aminofoamer FLDS-L (25 % Sodium Lauroyl Aspartate-containing fluid (Asahi Kasei Chemicals))

*2 : Aminosurfact ACMT-L (30 % Sodium Cocoyl Glutamate-containing fluid (Asahi Kasei Chemicals))

*3 : DIAPON K-SF (30 % Sodium Methyl Cocoyl Taurate-containing fluid (NOF Corporation))

*4 : Jordapon CI P (BASF Japan Ltd.)

*5 : Lipolan LJ-441 (about 35 % Sodium Tetradecenesulfonate-containing fluid (LION Corporation))

*6: Softazoline CH-R (30 % Alkyl Carboxymethyl Hydroxyethyl Imidazolinium Betaine-containing fluid (Kawaken Fine Chemicals))

*7 : Softazoline LSB-R (about 30 % Lauramidopropyl Hydroxysultaine-containing fluid (Kawaken Fine Chemicals))

*8: Viscosafe LPE (Kawaken Fine Chemicals)

*9 : Rheodol TW-IS399c (Kao Corporation)

The feelings in use showed in Table 1 were evaluated on the basis of the irritation of scalp in cleaning head hair, the smoothness in finger-combing in rinsing away, and the dry feeling of scalp/head hair after use.

Results are showed as follows:

Feeling in use was unsatisfying: Δ;
Feeling in use was satisfying: ○.

The cleaning formulations were prepared by a conventional procedure.

The results showed that the use of 5 % by weight or more of an acylamino acid type surfactant having an acidic amino acid moiety is preferable to improve the feeling in use, in particular, an amino acid such as aspartic acid and glutamic acid is more preferable.

Test example 2: Test of Viscosity

In order to improve the low viscosity of a cleaning formulation comprising an acylamino acid type surfactant, the viscosity change in the combination of an acylamino acid type surfactant, an ampholytic surfactant and/or a non-ionic surfactant was examined.

Method to determine viscosity:

The viscosity of the composition of the present invention was determined with a B type viscometer (BL type (TOKI SANGYO)). Rotor No. 2 was used for a composition with a low viscosity (<400 mPa · s) and rotor No. 3 was used for a composition with a relatively high viscosity. The viscosity was determined at 20°C at a rate of 12 or 30 rpm according to a conventional way.

Sodium lauroyl aspartate and triethanolamine-cocoyl glutamate were mixed in equal amount to give a mixture as an acylamino acid type surfactant. Then, in order to find a suitable combination of an ampholytic surfactant and a non-ionic surfactant for improving the low viscosity of an acylamino acid type surfactant, the amino acid type surfactant mixture, a non-ionic surfactant, and an ampholytic surfactant were mixed, wherein the final concentration of a non-ionic surfactant was 3 % by weight, and the final concentration of a combination of the amino acid type surfactant mixture and an ampholytic surfactant was 15 % by weight. Water was added to the resulting mixture to give a composition comprising three different surfactants (i.e., said composition was a mixture of the acylamino acid type surfactant mixture, a non-ionic surfactant, an ampholytic surfactant and water), and then the viscosity changes thereof were examined.

Table 2 indicates ampholytic surfactants and non-ionic surfactants used herein. As an ampholytic surfactant, lauramidopropyl betaine, lauramidopropyl hydroxysultaine, alkyl carboxymethyl hydroxyethyl imidazolinium betaine, and laurylaminodiacetate are used. As a non-ionic surfactant, butylene glycol laurate, lauryl glycol hydroxypropyl ether, and cocamide methy MEA are used.

The term "the viscosity change" used herein means the increase/decrease in the viscosity of a composition comprising three different surfactants (i.e., said composition is a mixture of the acylamino acid type surfactant mixture, a non-ionic surfactant, an ampholytic surfactant and water) compared with the viscosity of a composition comprising the acylamino acid type surfactant mixture (i.e., said composition is a mixture of 15 % by weight of the acylamino acid type surfactant mixture and water).

The compositions tested as to the viscosity comprised 3 % by weight of a non-ionic surfactant (butylene glycol laurate, lauryl glycol hydroxypropyl ether, or cocamide methy MEA), and 15 % by weight of a combination of the amino acid type surfactant mixture and an ampholytic surfactant.

The results are shown in Table 2.

Table 2

| | Content * | Butylene Glycol Laurate *5 | Lauryl Glycol Hydroxypropyl Ether *6 | Cocamide Methy MEA *7 |
|---|---|---|---|---|
| Lauramidopropyl Betaine *1 | 6-9 % | + | + | - |
| | 9-12 % | ++ | ++ | + |
| Lauramidopropyl Hydroxysultaine *2 | 6-9 % | - | - | - |
| | 9-12 % | +++ | +++ | + |
| Alkyl Carboxymethyl Hydroxyethyl Imidazolinium Betaine *3 | 6-9 % | - | + | - |
| | 9-12 % | + | ++ | ++ |
| Laurylaminodia cetate *4 | 6-9 % | - | - | - |
| | 9-12 % | - | - | - |

* "Content" means the amount (% by weight) of an ampholytic surfactant in a composition. The total amount of the mixture of acylamino acid type surfactants and an ampholytic surfactant is 15 % by weight of a composition.

*1: Amphorex LB-2 (about 30 % by weight Lauramidopropyl Betaine-containing fluid (MIYOSHI OIL & FAT CO., LTD.))

*2: Softazoline LSB-R (about 30 % by weight Lauramidopropyl Hydroxysultaine-containing fluid (Kawaken Fine Chemicals))

*3: Lebon CIB (about 35 % by weight Alkyl Carboxymethyl Hydroxyethyl Imidazolinium Betaine-containing fluid (Sanyo Chemical Industries, Ltd.))

*4: Nissan Anon LA (about 30 % by weight Laurylaminodiacetate-containing fluid (NOF CORPORATION))

*5: Compol BL (NOF CORPORATION)

*6: Viscosafe LPE (Kawaken Fine Chemicals)

*7: Aminon C-11S (Kao Corporation)

In Table 2:

the symbol "-" denotes no change of the viscosity;
the symbol "+" denotes an increase in the viscosity (to 200 mPa · s) ;
the symbol "++" denotes a large increase in the viscosity (200 to 1000 mPa · s);
the symbol "+++" denotes a very large increase in the viscosity (> 1000 mPa · s)

The results showed that the low viscosity of a composition comprising an acylamino acid type surfactant is improved by adding an ampholytic surfactant having a betaine skeleton besides an acylamino acid type surfactant and a non-ionic surfactant, and thereby the viscosity of the composition was increased.

Additionally, the compositions whose viscosity was enhanced in the above viscosity test also showed an improved quality of foam, an improved durability of foam, and an improved feeling in use, when the compositions were used in cleaning.

Test example 3: Viscosity change in relation to the concentration of non-ionic surfactant

In order to evaluate the viscosity change of a composition comprising an acylamino acid type surfactant in relation to the amount of a non-ionic surfactant, Test compositions 1 to 4 as shown in table 3 were prepared, and the viscosity changes were examined. POE glyceryl monococoate was used as a non-ionic surfactant (the amounts in the table are represented by a percentage by weight).

Table 3

|  | Test Composition 1 | Test Composition 2 | Test Composition 3 | Test Composition 4 |
|---|---|---|---|---|
| Sodium Lauroyl Aspartate | 8.5 | 8.5 | 8.5 | 8.5 |
| Lauramidopropyl Betaine *1 | 4.5 | 4.5 | 4.5 | 4.5 |
| Sodium Tetradecenesulfonate | 2 | 2 | 2 | 2 |
| POE Glyceryl Monococoate *2 | 0 | 2 | 3 | 5 |
| Cationic Polymer | 3 | 3 | 3 | 3 |
| Bactericidal Agent · Preservative Agent · Anti-Inflammatory Agent · Moisturizing Agent | Suitable Amount | Suitable Amount | Suitable Amount | Suitable Amount |
| Solvent · pH Adjuster · Flavor · Algefacient | Suitable Amount | Suitable Amount | Suitable Amount | Suitable Amount |
| Purified Water | Balance | Balance | Balance | Balance |
| Total | 100 | 100 | 100 | 100 |
| Viscosity (mPa · s) | 125 | 575 | 600 | 725 |
| *1: Nissan Anon BDL-SF (30 % Lauramidopropyl Betaine-containing fluid (NOF Corporation)) | | | | |
| *2: Glycerox HE (Croda Japan KK) | | | | |

The viscosity was determined according to the method of Test example 2. The results showed that a non-ionic surfactant is necessary to increase the viscosity of a composition. The results showed that Test compositions 2-4 had a viscosity of 300 mPa · s or more, and hence they have a suitable viscosity for using as an shampoo and the like.

Test example 4: Improvement of quick-foaming property

As for compositions tested in Test examples 1 to 3, the change in the lather of a composition comprising an anionic surfactant was examined in order to improve the lather, especially improve the quick-foaming property.

In order to measure the volume of a quickly-occurring foam, a control composition and Test compositions 1-5 were prepared as shown in Table 4 (the amounts in the table are represented by a percentage by weight). Then, each of the control composition and Test compositions 1-5 was diluted by 7-fold with purified water, and the resulting dilutions were stirred with fiberMixer (MX-X58 (Panasonic)) at a slow speed mode at room temperature for 5 seconds. The foam volume (mL) obtained shortly after stirring was treated as a volume of a quickly-occurring foam, and the differences between the foam volumes of the control composition and the test compositions were evaluated.

Table 4

| | Control Composition | Test Composition 1 | Test Composition 2 | Test Composition 3 | Test Composition 4 | Test Composition 5 |
|---|---|---|---|---|---|---|
| Sodium Lauroyl Aspartate | 8 | 10 | 8 | 8 | 8 | 8 |
| Alkyl Carboxymethyl Hydroxyethyl | 3 | 3 | 3 | 3 | 3 | 3 |
| Imidazolinium Betaine | | | | | | |
| POE Glyceryl Monococoate | 2 | 2 | 2 | 2 | 2 | 2 |
| Sodium Cocoyl Glutamate | | | 2 | | | |
| Sodium Methyl Cocoyl Taurate | | | | 2 | | |
| Sodium Lauroyl Methylaminopropionate *1 | | | | | 2 | |
| Sodium Tetradecenesulfonate | | | | | | 2 |
| pH adjuster | Suitable Amount | Suitable Amount | Suitable Amount | Suitable Amount | Suitable Amount | Suitable Amount |
| Purified Water | Balance | Balance | Balance | Balance | Balance | Balance |
| Total | 100 | 100 | 100 | 100 | 100 | 100 |
| pH | 6.5 | 6.5 | 6.5 | 6.5 | 6.5 | 6.5 |
| Foam Volume (mL) | 533 | 562 | 557 | 593 | 593 | 630 |
| Δ value (Test composition - Control composition) | 0 | 28 | 23 | 60 | 60 | 97 |
| *1: Alanon ALE (30 % by weight Sodium Lauroyl Methylaminopropionate-containing fluid (Kawaken Fine Chemicals)) | | | | | | |

EP 2 612 653 B2

14

The results showed that there was little increase in the foam volumes of Test compositions 1 and 2 comprising the larger amount of an acylamino acid type surfactant having an aspartate moiety or a glutamate moiety than that of the control composition. In contrast, Test compositions 3 to 5 which further comprised a different type of anionic surfactant showed a large increase in the foam volume and the improved quick-foaming property.

Test example 5-1: Formation of Coacervate

On the basis of the results of Test examples 1 to 4, the influence in the coacervate formation of each composition was evaluated about the addition of a cationic polymer.

Washing/cleaning liquids 1 to 4 which comprised different amounts of cationic cellulose 1 which is a cationic polymer were prepared as showed in Table 5 (the amounts in the table are represented by a percentage by weight). Each of Washing liquids 1 to 4 was diluted with purified water, and then transmissions (%) of each of the resulting dilutions were measured with an ultraviolet-visible spectrophotometer (UV-1800 (Shimadzu corporation); 420 nm) at 40°C. The turbidity (%) was calculated by the following equation using the obtained transmissions, and the turbidity was used as an index of the amount of the coacervate formation to evaluate the coacervate formation.

$$\text{Turbidity (\%)} = 100 - \text{Transmission (\%)}$$

Table 5

|  | Washing Liquid 1 | Washing Liquid 2 | Washing Liquid 3 | Washing Liquid 4 |
|---|---|---|---|---|
| Sodium Lauroyl Aspartate | 8.5 | 8.5 | 8.5 | 8.5 |
| Lauramidopropyl Betaine | 4.5 | 4.5 | 4.5 | 4.5 |
| Sodium Tetradecenesulfonate | 2 | 2 | 2 | 2 |
| POE Glyceryl Monococoate | 2 | 2 | 2 | 2 |
| Cationic Cellulose 1 *1 | 0.1 | 0.5 | 2 | 3 |
| Solvent · | Suitable | Suitable | Suitable | Suitable |
| Preservative Agent · pH Adjuster | Amount | Amount | Amount | Amount |
| Purified Water | Balance | Balance | Balance | Balance |
| Total | 100 | 100 | 100 | 100 |
| *1: MERQUAT 10 (Nalco Japan) | | | | |

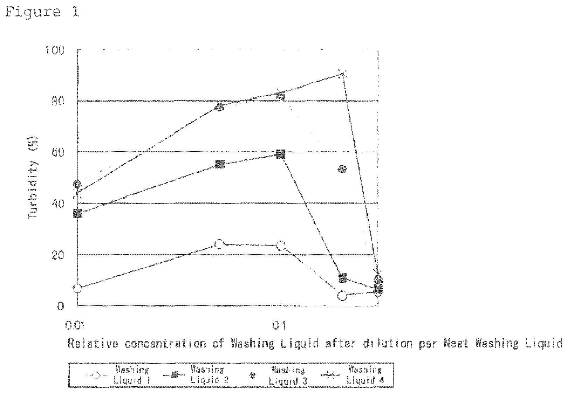

As showed in Figure 1, there was little increase in the turbidity of the dilutions of Washing liquid 1, and the amount of a coacervate formation was small. In contrast, there were sufficient increases in the turbidity of the dilutions of Washing liquids 2 to 4. This shows that Washing liquids 2 to 4 may form the coacervate in cleaning or in rinsing away, and thereby they may provide an excellent conditioning benefit when applied to head hair as a shampoo.

Test example 5-2

Additionally, in order to evaluate the coacervate formation in relation to the difference of polymerization degrees of cationic cellulose, and in relation to adding other cationic polymers, Washing liquids 5 to 9 were prepared as shown in Table 6, and the change in the amount of the coacervate formation was evaluated by measuring the turbidity change in a manner similar to Test example 5-1 (the amounts of in the table are represented by a percentage by weight).

Table 6

|  | Washing Liquid 5 | Washing Liquid 6 | Washing Liquid 7 | Washing Liquid 8 | Washing Liquid 9 |
|---|---|---|---|---|---|
| Sodium Lauroyl Aspartate | 8.5 | 8.5 | 8.5 | 8.5 | 8.5 |
| Lauramidopropyl Betaine | 4.5 | 4.5 | 4.5 | 4.5 | 4.5 |

(continued)

|  | Washing Liquid 5 | Washing Liquid 6 | Washing Liquid 7 | Washing Liquid 8 | Washing Liquid 9 |
|---|---|---|---|---|---|
| Sodium Tetradecenesulfonate | 2 | 2 | 2 | 2 | 2 |
| POE Glyceryl Monococoate | 2 | 2 | 2 | 2 | 2 |
| Cationic Cellulose 1 | 0.1 | 0.4 | 0.7 | 1.4 | 2.1 |
| Cationic Cellulose 2 *2 | 0.0 | 0.2 | 0.3 | 0.6 | 0.9 |
| Cationic Polymer *3 | 2 | 2 | 2 | 2 | 2 |
| pH Adjuster, Preservative Agent, Solvent | Suitable Amount | Suitable Amount | Suitable Amount | Suitable Amount | Suitable Amount |
| Purified Water | Balance | Balance | Balance | Balance | Balance |
| Total | 100 | 100 | 100 | 100 | 100 |
| *2: CELQUAT SC-230M (Akzo Nobel) *3: MERQUAT 550PR (Nalco Japan) | | | | | |

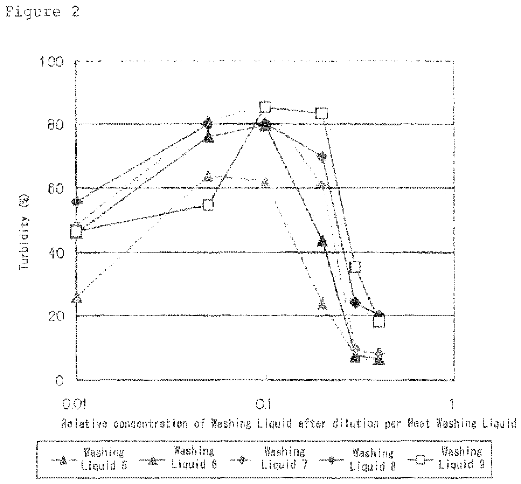

As showed in Figure 2, Washing liquids 5 to 8 comprising cationic celluloses which have different polymerization degrees and/or another cationic polymer also showed high turbidity. On the other hand, the coacervate formation was confirmed in Washing liquid 9, but the dilution of Washing liquid 9 was heterogeneous due to clumping.

Therefore, it was shown that the coacervate formation of the composition of the present invention was improved by adding cationic cellulose or a cationic polymer.

Test example 6

In order to evaluate the actual effect of the composition of the present invention on hair, the following test was conducted.

A bundle of human hair was bleached and permed, and then ultra-sonicated to give a damaged hair. Washing liquid 10 (0.2 g) as showed in Table 7 (the amounts in the table are represented by a percentage by weight) was applied to the damaged hair, and then the hair was lathered up for 1 minute, and the Washing liquid was removed.

Washing liquid 10 (0.1 g) was again applied to the hair uniformly, and the hair was lathered up for 1 minute, and then the washing liquid was removed, and the hair was dried. In order to evaluate the change in the smoothness of hair after the above treatment, the difference of frictions between before and after the treatment was measured with Friction Tester KES-SE-DC (KATO TECH).

The friction tester is a device used to assess the feeling in touching an object, such as smoothness and rough texture. The rough texture is quantified as a variation of mean friction coefficient (MMD). The lower MMD is, the smoother the surface of the object is.

Table 7

| Washing Liquid 10 | |
|---|---|
| Sodium Lauroyl Aspartate | 8.5 |
| Lauramidopropyl Betaine | 4.5 |
| POE Glyceryl Monococoate | 2 |
| Sodium Tetradecenesulfonate | 2.1 |
| Cationic Cellulose | 1 |
| Cationic Polymer | 2 |
| Bactericidal Agent · preservative Agent · Anti-Inflammatory Agent · Moisturizing Agent | Suitable Amount |
| Solvent · pH Adjuster · Flavor | Suitable Amount |
| Algefacient | 0.3 |

(continued)

| Washing Liquid 10 | |
|---|---|
| Purified Water | Balance |
| Total | 100 |

Table 8

| | Damaged hair bundle | Hair bundle after the treatment |
|---|---|---|
| MMD of the hair bundle after the treatment is shown, as compared to MMD of the damaged hair bundle without the treatment which is taken as 100 %. | 100 | 56 |

As showed in Table 8, the hair bundle after the treatment with the washing liquid became smoother than the damaged hair bundle without the treatment.

This result showed that the damaged hair became smoother by the treatment with Washing liquid 10. It is thought that the treatment with Washing liquid 10 leads to a coacervate formation on hair, and thereby the texture of damaged hair is improved.

Test example 7

In order to evaluate the effect of the composition of the present invention on a hair surface, the structural change of a treated hair was observed.

A bundle of human hair was treated with a commercially available decoloring agent and a commercially available hairdye to give a damaged hair as a sample before treatment. The damaged hair was soaked in 10 % Washing liquid 10 at 50°C for 1 minute, and rinsed, and then dried to give a sample after treatment. The samples before and after treatment were observed with a scanning electron microscope (JSM-6380LV(JEOL Ltd.)) to evaluate the change of the surface of hair before and after treatment.

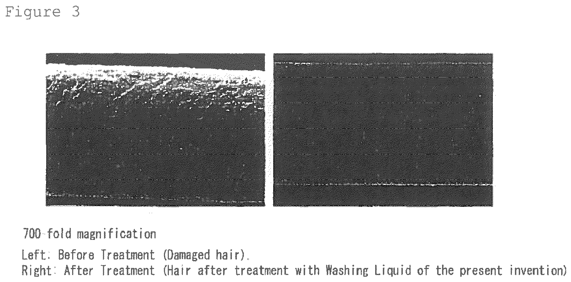

As showed in Figure 3, the damaged hair before treatment had many damaged areas in the hair surface. In contrast, the damaged areas were reduced in the sample after treatment, and it was confirmed by such structural observation that the surface of hair became smoother.

The following formulation for cleaning scalp/head hair which has a high transparency may be prepared.

Table 9

| Formulation example | |
|---|---|
| Triethanolamine-cocoyl glutamate | 2.4 (% by weight) |
| Sodium Lauroyl Aspartate | 7.5 |
| Sodium Methyl Caproyl Taurate | 3.6 |
| Sodium Olefin Sulfonate (Sodium Tetradecenesulfonate) | 2.1 |
| Alkyl Carboxymethyl Hydroxyethyl Imidazolinium Betaine | 4.5 |
| Lauryl Glycol Hydroxypropyl Ether | 3 |
| Dipropylene Glycol | 2 |
| [Hydroxy(Trimethylammonio)Propyl]Hydroxyethylcellulose Chloride | 1 |
| Dimethyldiallyl Ammonium Chloride · Acrylamide Copolymer | 0.2 |
| Preservative Agent · pH Adjuster | Suitable Amount |
| Purified Water | Balance |
| Total | 100 |
| (Viscosity) | 2640 mPa · s |

**Claims**

1. A cleaning composition applicable to scalp and head hair which comprises
Ingredient (a): an acylamino acid type surfactant 7.5 to 12 % by weight;
Ingredient (b): a betaine-type ampholytic surfactant; and
Ingredient (c): a non-ionic surfactant 1.5 to 10 % by weight,
wherein the acylamino acid type surfactant of Ingredient (a) is a compound represented by the formula:

$$R\text{-}NH\text{-}CH(COOX_1)((CH_2)_nCOOX_2)$$

wherein

R is an acyl group having 8 to 20 carbon atoms;
n is 1; and
$X_1$ and $X_2$ are each independently a hydrogen atom, an alkali metal, an alkaline earth metal, an ammonium group, or a cationic residue of a basic amino acid or an alkanolamine;
wherein the betaine-type ampholytic surfactant of Ingredient (b) is amide carboxy betaine;
wherein the non-ionic surfactant of Ingredient (c) is selected from POE glycerin fatty acid ester, POE sorbitan fatty acid ester, POE sorbitol fatty acid ester, ethylene glycol fatty acid ester, propylene glycol fatty acid ester, butylene glycol fatty acid ester, pentaerythritol fatty acid ester, and an ether type non-ionic surfactant which is in the form of a condensation product of alkyl glycol and propylene glycol, wherein the amount of Ingredient (b) is from 0.4 to 1 times by weight as much as the amount of Ingredient (a);
wherein the amount of Ingredient (b) is from 1.5 to 3 times by weight as much as the amount of Ingredient (c);
wherein the amount of Ingredient (c) is from 0.2 to 0.5 times by weight as much as the amount of Ingredient (a), and
wherein the cleaning composition has a viscosity ranging from 300 to 4000 mPa·s at 20 °C.

2. The cleaning composition according to claim 1, wherein R is selected from a capryloyl group, a caprinoyl group, a lauroyl group, a myristoyl group, a palmitoyl group, a stearoyl group, an oleoyl group, a cocoyl group, an acyl group derived from palm kernel oil fatty acid, and an acyl group derived from hydrogenated tallow fatty acid.

3. The cleaning composition according to claim 1 or 2, wherein the acylamino acid type surfactant of Ingredient (a) is lauroyl aspartic acid or a salt thereof.

4. The cleaning composition according to any one of claims 1-3, which comprises 2 to 5 % by weight of the non-ionic surfactant of Ingredient (c).

5. The cleaning composition according to any one of claims 1-4, which further comprises Ingredient (d): a cationic polymer, wherein the amount of Ingredient (d) is from 0.025 to 1 times by weight as much as the amount of Ingredient (a).

6. The cleaning composition according to claim 5, wherein the cationic polymer of Ingredient (d) comprises cationic cellulose and/or dimethyldiallyl ammonium chloride-acrylamide copolymer.

7. The cleaning composition according to any one of claims 1-6, which further comprises
Ingredient (e): an anionic surfactant other than an acylamino acid type surfactant 0.1 to 5 % by weight
wherein said acylamino acid type surfactant is a compound represented by the formula:

$$R\text{-}NH\text{-}CH(COOX_1)((CH_2)_nCOOX_2)$$

wherein

R is an acyl group having 8 to 20 carbon atoms;
n is 1 or 2; and
$X_1$ and $X_2$ are each independently a hydrogen atom, an alkali metal, an alkaline earth metal, an ammonium group, or a cationic residue of a basic amino acid or an alkanolamine.

8. The cleaning composition according to claim 7 wherein the anionic surfactant of Ingredient (e) is selected from:

an anionic surfactant which has an acyl group having 6 to 20 carbon atoms and a taurine skeleton;
an anionic surfactant which has an acyl group having 6 to 20 carbon atoms and an alanine skeleton; and
an anionic surfactant which is a sulfonic acid compound having an alkyl group and/or an alkenyl group each of which has 10 to 20 carbon atoms, or a salt thereof.

9. The cleaning composition according to claim 1 which further comprises
Ingredient (d): a cationic polymer; and
Ingredient (e): an anionic surfactant other than an acylamino acid type surfactant which is a compound represented by the formula:

$$R-NH-CH(COOX_1)((CH_2)_nCOOX_2)$$

wherein

R is an acyl group having 8 to 20 carbon atoms;
n is 1 or 2; and
$X_1$ and $X_2$ are each independently a hydrogen atom, an alkali metal, an alkaline earth metal, an ammonium group, or a cationic residue of a basic amino acid or an alkanolamine,
wherein
the amount of Ingredient (d) is from 0.025 to 1 times by weight as much as the amount of Ingredient (a); and
the amount of Ingredient (e) is from 0.01 to 0.6 times by weight as much as the amount of Ingredient (a).

10. The composition for cleaning scalp and head hair according to claim 9, wherein a coacervate is formed when the composition is diluted by 5 to 100-fold with water.

11. The cleaning composition according to claim 1, which further comprises
Ingredient (e): an anionic surfactant 1 to 4 % by weight,
wherein the anionic surfactant of Ingredient (e) is selected from:

an anionic surfactant which has an acyl group having 6 to 20 carbon atoms and a taurine skeleton;
an anionic surfactant which has an acyl group having 6 to 20 carbon atoms and an alanine skeleton; and
an anionic surfactant which is a sulfonic acid compound having an alkyl group and/or an alkenyl group each of which has 10 to 20 carbon atoms, or a salt thereof,
wherein
the amount of Ingredient (b) is from 2 to 15 % by weight; and
the amount of Ingredient (e) is from 0.1 to 0.5 times by weight as much as the amount of Ingredient (a).

**Patentansprüche**

1. Reinigungszusammensetzung anwendbar auf Kopfhaut und Kopfhaar, die umfasst:

Inhaltsstoff (a): ein oberflächenaktives Mittel vom Acylaminosäuretyp, 7,5 bis 12 Gewichts-%;
Inhaltsstoff (b): ein ampholytisches oberflächenaktives Mittel vom Betaintyp; und
Inhaltsstoff (c): ein nichtionisches oberflächenaktives Mittel, 1,5 bis 10 Gewichts-%,

wobei das oberflächenaktive Mittel vom Acylaminosäuretyp des Inhaltsstoffs (a) eine Verbindung ist, die durch die Formel:

$$R-NH-CH(COOX_1)((CH_2)_nCOOX_2)$$

dargestellt wird, wobei

R eine Acylgruppe mit 8 bis 20 Kohlenstoffatomen ist;
n 1 ist; und
$X_1$ und $X_2$ jeweils unabhängig ein Wasserstoffatom, ein Alkalimetall, ein Erdalkalimetall, eine Ammoniumgruppe oder ein kationischer Rest einer basischen Aminosäure oder eines Alkanolamins sind,

wobei das ampholytische oberflächenaktive Mittel vom Betaintyp des Inhaltsstoffs (b) Amidcarboxy-Betain ist; wobei das nichtionische oberflächenaktive Mittel des Inhaltsstoffs (c) aus POE-Glycerinfettsäureester, POE-Sorbitanfettsäureester, POE-Sorbitfettsäureester, Ethylenglycolfettsäureester, Propylenglycolfettsäureester, Butylenglycolfettsäureester, Pentaerythritfettsäureester und einem nichtionischen oberflächenaktiven Mittel des Ethertyps, das in Form eines Kondensationsprodukts von Alkylglycol und Propylenglycol vorliegt, ausgewählt ist; wobei die Menge des Inhaltsstoffs (b) von 0,4- bis 1-mal soviel wie die Menge des Inhaltsstoffs (a) beträgt, bezogen auf das Gewicht; wobei die Menge des Inhaltsstoffs (b) von 1,5- bis 3-mal soviel wie die Menge des Inhaltsstoffs (c) beträgt, bezogen auf das Gewicht; wobei die Menge des Inhaltsstoffs (c) von 0,2- bis 0,5-mal soviel wie die Menge des Inhaltsstoffs (a) beträgt, bezogen auf das Gewicht; und wobei die Reinigungszusammensetzung bei 20 °C eine Viskosität im Bereich von 300 bis 4000 mPa·s aufweist.

2. Reinigungszusammensetzung nach Anspruch 1, wobei R ausgewählt ist aus einer Capryloylgruppe, einer Caprinoylgruppe, einer Lauroylgruppe, einer Myristoylgruppe, einer Palmitoylgruppe, einer Stearoylgruppe, einer Oleoylgruppe, einer Cocoylgruppe, einer Acylgruppe abgeleitet von Palmkernölfettsäure, und einer Acylgruppe abgeleitet aus hydrogenierter Talgfettsäure.

3. Reinigungszusammensetzung nach Anspruch 1 oder 2, wobei das oberflächenaktive Mittel vom Acylaminosäuretyp des Inhaltsstoffs (a) Lauroylasparaginsäure oder ein Salz davon ist.

4. Reinigungszusammensetzung nach einem der Ansprüche 1-3, das 2 bis 5 Gewichts-% des nichtionischen oberflächenaktiven Mittels von Inhaltsstoff (c) umfasst.

5. Reinigungszusammensetzung nach einem der Ansprüche 1-4, die weiterhin Inhaltsstoff (d) umfasst: ein kationisches Polymer, wobei die Menge des Inhaltsstoffs (d) von 0,025- bis 1-mal soviel wie die Menge des Inhaltsstoffs (a) beträgt, bezogen auf das Gewicht.

6. Reinigungszusammensetzung nach Anspruch 5, wobei das kationische Polymer von Inhaltsstoff (d) kationische Cellulose und/oder Dimethyldiallylammoniumchlorid-Acrylamid-Copolymer umfasst.

7. Reinigungszusammensetzung nach einem der Ansprüche 1-6, die weiter umfasst: Inhaltsstoff (e): ein anionisches oberflächenaktives Mittel außer einem oberflächenaktiven Mittel vom Acylaminosäuretyp, 0,1 bis 5 Gewichts-%, wobei das oberflächenaktive Mittel vom Acylaminosäuretyp eine Verbindung ist, die durch die Formel:

$$R\text{-}NH\text{-}CH(COOX_1)((CH_2)_n COOX_2)$$

dargestellt wird, wobei

R eine Acylgruppe mit 8 bis 20 Kohlenstoffatomen ist; n 1 oder 2 ist; und $X_1$ und $X_2$ jeweils unabhängig ein Wasserstoffatom, ein Alkalimetall, ein Erdalkalimetall, eine Ammoniumgruppe, oder ein kationischer Rest einer basischen Aminosäure oder eines Alkanolamins sind.

8. Reinigungszusammensetzung nach Anspruch 7, wobei das anionische oberflächenaktive Mittel von Inhaltsstoff (e) ausgewählt ist aus:

einem anionischen oberflächenaktiven Mittel, das eine Acylgruppe mit 6 bis 20 Kohlenstoffatomen und ein Taurinskelett aufweist; einem anionischen oberflächenaktiven Mittel, das eine Acylgruppe mit 6 bis 20 Kohlenstoffatomen und ein Alaninskelett aufweist; und einem anionischen oberflächenaktiven Mittel, das eine Sulfonsäureverbindung mit einer Alkylgruppe und/oder einer Alkenylgruppe, die jeweils 10 bis 20 Kohlenstoffatome aufweisen, oder ein Salz davon ist.

9. Reinigungszusammensetzung nach Anspruch 1, die weiter umfasst:

Inhaltsstoff (d): ein kationisches Polymer; und

Inhaltsstoff (e): ein anionisches oberflächenaktives Mittel außer einem oberflächenaktiven Mittel vom Acylaminosäuretyp, das eine Verbindung ist, die durch die Formel:

$$R\text{-}NH\text{-}CH(COOX_1)((CH_2)_nCOOX_2)$$

dargestellt wird, wobei

R eine Acylgruppe mit 8 bis 20 Kohlenstoffatomen ist;
n 1 oder 2 ist; und
$X_1$ und $X_2$ jeweils unabhängig ein Wasserstoffatom, ein Alkalimetall, ein Erdalkalimetall, eine Ammoniumgruppe, oder ein kationischer Rest einer basischen Aminosäure oder eines Alkanolamins sind,

wobei
die Menge von Inhaltsstoff (d) von 0,025- bis 1-mal soviel wie die Menge von Inhaltsstoff (a) beträgt, bezogen auf das Gewicht; und
die Menge von Inhaltsstoff (e) von 0,01- bis 0,6-mal soviel wie die Menge von Inhaltsstoff (a) beträgt, bezogen auf das Gewicht.

10. Zusammensetzung zur Reinigung von Kopfhaut und Kopfhaar nach Anspruch 9, wobei ein Coazervat gebildet wird, wenn die Zusammensetzung 5- bis 100-fach mit Wasser verdünnt wird.

11. Reinigungszusammensetzung nach Anspruch 1, die weiter umfasst:

Inhaltsstoff (e): ein anionisches oberflächenaktives Mittel, 1 bis 4 Gewichts-%,
wobei das anionische oberflächenaktive Mittel des Inhaltsstoffs (e) ausgewählt ist aus:

einem anionischen oberflächenaktiven Mittel, das eine Acylgruppe mit 6 bis 20 Kohlenstoffatomen und ein Taurinskelett aufweist;
einem anionischen oberflächenaktiven Mittel, das eine Acylgruppe mit 6 bis 20 Kohlenstoffatomen und ein Alaninskelett aufweist; und
einem anionischen oberflächenaktiven Mittel, das eine Sulfonsäureverbindung mit einer Alkylgruppe und/oder einer Alkenylgruppe, die jeweils 10 bis 20 Kohlenstoffatome aufweisen, oder ein Salz davon ist,

wobei
die Menge von Inhaltsstoff (b) im Bereich von 2 bis 15 Gewichts-% liegt; und
die Menge von Inhaltsstoff (e) von 0,1- bis 0,5-mal soviel wie die Menge von Inhaltsstoff (a) beträgt, bezogen auf das Gewicht.

**Revendications**

1. Composition nettoyante pouvant être appliquée sur le cuir chevelu et les cheveux, comprenant
un ingrédient (a) : un agent tensioactif de type acide aminé acylé, de 7,5 à 12 % en poids ;
un ingrédient (b) : un agent tensioactif ampholyte de type bétaïne ; et
un ingrédient (c) : un agent tensioactif non ionique, de 1,5 à 10 % en poids ; où l'agent tensioactif de type acide aminé acylé de l'ingrédient (a) est un composé représenté par la formule :

$$R\text{-}NH\text{-}CH(COOX_1)((CH_2)_nCOOX_2)$$

dans laquelle

R est un groupement acyle comportant 8 à 20 atomes de carbone ;
n vaut 1 ; et
$X_1$ et $X_2$ sont chacun indépendamment un atome d'hydrogène, un métal alcalin, un métal alcalino-terreux, un groupement ammonium, ou un résidu cationique d'un acide aminé basique ou une alcanolamine ;

où le tensioactif ampholyte de type bétaïne de l'ingrédient (b) est une carboxybétaïne amidée ;
où le tensioactif non ionique de l'ingrédient (c) est sélectionné dans le groupe composé d'un ester d'acide gras de POE-glycérine, d'un ester d'acide gras de POE-sorbitane, d'un ester d'acide gras de POE-sorbitol, d'un ester d'acide gras d'éthylèneglycol, d'un ester d'acide gras de propylèneglycol, d'un ester d'acide gras de butylèneglycol, d'un ester d'acide gras de pentaérythritol, et d'un tensioactif non ionique de type éther qui se présente sous la forme d'un produit de condensation d'alkylglycol et de propylèneglycol,

où la quantité de l'ingrédient (b) correspond à 0,4 à 1 fois en poids celle de l'ingrédient (a) ;
où la quantité de l'ingrédient (b) correspond à 1,5 à 3 fois en poids celle de l'ingrédient (c) ; et
où la quantité de l'ingrédient (c) correspond à 0,2 à 0,5 fois en poids celle de l'ingrédient (a) ; et
où la composition nettoyante possède une viscosité allant de 300 à 4000 mPa.s à 20°C.

2. Composition nettoyante selon la revendication 1, dans laquelle R est sélectionné parmi un groupement capryloyle, un groupement caprinoyle, un groupement lauroyle, un groupement myristoyle, un groupement palmitoyle, un groupement stéaroyle, un groupement oléoyle, un groupement cocoyle, un groupement acyle dérivé d'un acide gras d'huile de palmiste, et un groupement acyle dérivé d'un acide gras de suif hydrogéné.

3. Composition nettoyante selon la revendication 1 ou 2, dans laquelle l'agent tensioactif de type acide aminé acylé de l'ingrédient (a) est l'acide lauroyl-aspartique ou un sel de celui-ci.

4. Composition nettoyante selon l'une quelconque des revendications 1 à 3, comprenant 2 à 5 % en poids de l'agent tensioactif non ionique de l'ingrédient (c).

5. Composition nettoyante selon l'une quelconque des revendications 1 à 4, qui contient en outre l'ingrédient (d) : un polymère cationique, où la quantité de l'ingrédient (d) correspond à 0,025 à 1 fois en poids celle de l'ingrédient (a).

6. Composition nettoyante selon la revendication 5, dans laquelle le polymère cationique de l'ingrédient (d) comprend une cellulose cationique et/ou un copolymère de chlorure de diméthyldiallylammonium·acrylamide.

7. Composition nettoyante selon l'une quelconque des revendications 1 à 6, comprenant en outre
un ingrédient (e) : un agent tensioactif anionique autre qu'un agent tensioactif de type acide aminé acylé, de 0,1 à 5 % en poids ;
où ledit agent tensioactif de type acide aminé acylé est un composé représenté par la formule :

$$R\text{-}NH\text{-}CH(COOX_1)((CH_2)_nCOOX_2)$$

dans laquelle

R est un groupement acyle ayant de 8 à 20 atomes de carbone ;
n vaut 1 ou 2 ; et
$X_1$ et $X_2$ sont chacun indépendamment un atome d'hydrogène, un métal alcalin, un métal alcalino-terreux, un groupement ammonium, ou un résidu cationique d'un acide aminé basique ou une alcanolamine.

8. Composition nettoyante selon la revendication 7, dans laquelle l'agent tensioactif anionique de l'ingrédient (e) est sélectionné parmi :

un agent tensioactif anionique possédant un groupement acyle ayant 6 à 20 atomes de carbone et un squelette de taurine ;
un agent tensioactif anionique possédant un groupement acyle ayant 6 à 20 atomes de carbone et un squelette d'alanine ; et
un agent tensioactif anionique qui est un composé d'acide sulfonique possédant un groupement alkyle et/ou un groupement alcényle, chacun d'entre eux comportant 10 à 20 atomes de carbone, ou un sel de ceux-ci.

9. Composition nettoyante selon la revendication 1, comprenant en outre
un ingrédient (d) : un polymère cationique ; et
un ingrédient (e) : un agent tensioactif anionique autre qu'un agent tensioactif de type acide aminé acylé, qui est un composé représenté par la formule :

$$R\text{-}NH\text{-}CH(COOX_1)((CH_2)_nCOOX_2)$$

dans laquelle

R est un groupement acyle comportant 8 à 20 atomes de carbone ;

n vaut 1 ou 2 ; et

$X_1$ et $X_2$ sont chacun indépendamment un atome d'hydrogène, un métal alcalin, un métal alcalino-terreux, un groupement ammonium, ou un résidu cationique d'un acide aminé basique ou une alcanolamine,

où

la quantité d'ingrédient (d) correspond à 0,025 à 1 fois en poids celle de l'ingrédient (a) ; et

la quantité d'ingrédient (e) correspond à 0,01 à 0,6 fois en poids celle de l'ingrédient (a).

10. Composition destinée à nettoyer le cuir chevelu et les cheveux selon la revendication 9, dans laquelle un coacervat est formé lorsque la composition est diluée 5 à 100 fois avec de l'eau.

11. Composition nettoyante selon la revendication 1, comprenant en outre

un ingrédient (e) : un agent tensioactif anionique, de 1 à 4 % en poids ;

où l'agent tensioactif anionique de l'ingrédient (e) est sélectionné parmi :

un agent tensioactif anionique possédant un groupement acyle ayant 6 à 20 atomes de carbone et un squelette de taurine ;

un agent tensioactif anionique possédant un groupement acyle ayant 6 à 20 atomes de carbone et un squelette d'alanine ; et

un agent tensioactif anionique qui est un composé d'acide sulfonique possédant un groupement alkyle et/ou un groupement alcényle, chacun d'entre eux comportant 10 à 20 atomes de carbone, ou un sel de ceux-ci ;

où

la quantité d'ingrédient (b) va de 2 à 15 % en poids ; et

la quantité d'ingrédient (e) correspond à 0,1 à 0,5 fois en poids celle de l'ingrédient (a).

Figure 1

Relative concentration of Washing Liquid after dilution per Neat Washing Liquid

Washing Liquid 1   Washing Liquid 2   Washing Liquid 3   Washing Liquid 4

Figure 2

Relative concentration of Washing Liquid after dilution per Neat Washing Liquid

Figure 3

700-fold magnification

Left: Before Treatment (Damaged hair).
Right: After Treatment (Hair after treatment with Washing Liquid of the present invention)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005074868 A
- JP 2006306908 A
- JP 2006348101 A